# EUROPEAN PATENT SPECIFICATION

(11) **EP 1 115 344 B1**
(45) Date of publication and mention of the grant of the patent: **19.07.2006**
(21) Application number: 99948454.6
(22) Date of filing: 24.09.1999
(51) Int. Cl.: A61B 17/70

(54) **MULTI-AXIS CONNECTIONS BETWEEN SPINAL STABILIZERS AND SCREWS**
MEHRACHSIGE VERBINDUNGEN ZWISCHEN WIRBELSÄULENSTÜTZEN UND SCHRAUBEN
CONNEXIONS A PLUSIEURS AXES ENTRE STABILISATEURS VERTEBRAUX, ET VIS CORRESPONDANTES

(30) Priority: 25.09.1998 US 161141
(43) Date of publication of application: 18.07.2001
(73) Proprietor: Perumala Corporation, Brownsville, TX 78520-8817 (US)
(72) Inventor: PISHARODI, Madhavan, Brownsville, TX 78520 (US)
(74) Representative: Lockey, Robert Alexander
(86) International application number: PCT/US1999/022232
(87) International publication number: WO 2000/018312

(56) References cited:
- EP-A- 0 846 444
- FR-A- 2 683 445
- FR-A- 2 697 993
- FR-A- 2 735 011
- US-A- 5 092 893
- US-A- 5 344 421
- US-A- 5 531 747
- US-A- 5 743 907
- H.S.AN AND J.M.COTLER (EDS.): "Spinal Instrumentation" 1992 , WILLIAMS & WILKINS , BALTIMORE, US XP002128239 223960 cited in the application page 399 -page 400; figure 24.2

## Description

The present invention relates to apparatus of multi-axis internal spinal fixation. In more detail, the present invention relates to a connection for use in an internal spinal fixation system, and a method of stabilizing, or fixing, the spine for use with either bilateral rods or plates (such as the Steffee/variable screw placement system) or a central rod and plurality of cross-bars or plates (such as the so-called Tacoma Monorail System), utilizing wedge-shaped and/or flat washers having off set and/or centered openings therein to provide multiple axes for the pedicle screws used to fix the rods, cross-bars, and/or plates to the vertebrae of the patient, thereby effectively transferring the load from the spinal column to the spinal stabilizer.

There are many systems available for internal fixation of the spine. Such systems are described in the patent literature *(see,* for instance, U.S. Patent Nos. 4,696,290, 5,092,866, and 5,129,899) and the scientific literature *(see,* for instance, D.M Arnold and JE. Lonstein (Eds.), 6 State of the Art Reviews - Spine: Pedicle Fixation of the Lumbar Spine (Philadelphia: Nanley & Belfus, Inc.) 1992 and H.S. An and J.M. Cotler (Eds.), Spinal Instrumentation (Baltimore: Williams & Wilkins) 1992), and are available from such vendors as AcroMed, Smith & Nephew, MOSS® Miami, Osteonics, Sokmor Danek, and others.

A problem with all such systems, however, is the connection between the screws used to affix the system to the pedicle and the rods, cross-bars, and/or plates of the system. As stated in J.M Cotler, *et al.,* Principles, Indications, and Complications of Spinal Instrumentation: A Summary Chapter, in H.S. An and J. M Cotler, Spinal Instrumentation pp. 43 5-456 (Baltimore: Williams & Wilkins) 1992, "[a] significant problem in pedicular screw fixation appears to be at the site of linkage between the screw and rod or plate."

It appears that the problems at the site of this linkage may result from the geometry of the connection between the screw and the rod or plate. This difficult geometry results from several factors, including the different angles and placement of the vertebrae and their relative sizes, the shape of the vertebrae and the spacing between vertebrae, the placement of the screws, the lordosis of the spine, and the need to insert the screws into each vertebra at an angle. With regard to the angle of the pedicle screws, pedicle screws are angled inwardly and upwardly into the vertebra for maximum strength and, because the surfaces of the pedicles of each.vertebrae are angled relative to each other, the screws rarely line up across the vertebral body into which they are screwed. Nor do they usually line up from one vertebra to the adjacent vertebra, even if the adjacent vertebrae are the same size and shape (which they generally are not). For a more complete discussion of the biomechanics of the bone-implant interface, reference is made to H.A. Pool and R.W. Gaines, Biomechanics of Transpedicular Screw Spinal Implant Systems, *in* D.M Arnold and J.E. Lonstein (Eds.), 6 State of the Art Reviews - Spine: Pedicle Fixation of the Lumbar Spine 37-44 (Philadelphia: Nanley & Belfus, Inc.) 1992, M.R. Pinto, Complication of Pedicle Screw Fixation, in D.M. Arnold and J.E. Lonstein (Eds.), 6 State of the Art Reviews - Spine: Pedicle Fixation of the Lumbar Spine 45-54 (Philadelphia: Nanley & Belfus, Inc.) 1992, and M.H. Krag, Vermont Spinal Fixator, *in* D.M. Arnold and J.E. Lonstein (Eds.), 6 State of the Art Reviews - Spine: Pedicle Fixation of the Lumbar Spine 121-145 (Philadelphia: Nanley & Belfus, Inc.) 1992. Because the pedicle screws do not line up, the rod (or rods depending upon the particular stabilizer utilized) which runs along the longitudinal axis of the patient's spinal column, which provides the structural rigidity required to stabilize the spine, must either be bent to the location of each screw head or the stabilizer must be provided with adjustable structure which enables the head of the pedicle screw to be attached to the rod.

As a result of this difficulty, the literature includes comments such as the following statement in R.M. Puno and J.A. Byrd III, Transpedicular Screw/Rod Fixation Using the Puno-Winter-Byrd (PWB) System, in DM Arnold and J.E. Lonstein (Eds.), 6 State of the Art Reviews - Spine: Pedicle Fixation of the Lumbar Spine 83-106 (Philadelphia: Nanley & Belfus; Inc.) 1992:
"Transpedicular fixation has been proved to be of value in the treatment of spinal disorders.... However, experience has shown that this method of instrumentation places great demand on the surgeon's skill because of the anatomic constraints related mainly to the anatomy and morphometry of the spinal pedicle."

Many of the above-listed systems, and many of the systems described in the literature, attempt to relieve this burden on the surgeon by providing angled screws (for instance, the AMSET® R-F reduction-fixation system), so-called polyaxial screws (for example, the MOSS® Miami system noted above), full-length, scalloped, open-slot plate design with an undersurface complementary to the shape of the screw head (the Sofamor Danek plate and screw system noted above for example) for optimal positioning of the screws and up to 15° medial-lateral and 30° craniocaudal angulation at the screw-plate interface, and infinitely variable couplers (the so-called Rogozinski spinal rod system for example) which are said to allow rotation through a 130° arc to allow screw placement within the pedicle with no requirement to align each screw with the screw in the adjacent vertebrae.

Although these prior systems address these problems, as evidenced by the fact that new systems are introduced by the same vendors which are already marketing the above-listed systems, no currently available system completely solves all the problems presented by the need for optimal screw placement, angulation of the screw, and effective load transfer from spinal column to implant An ideal system would (a) accomodate optimal screw placement, height, and angulation, (b) accomodate different sizes and shapes of vertebrae, (c) minimize (or not require) bending or other fabrication during surgery, (d) maintain an angle of approximately 90° at the connection between the screw head and the plate or cross-bar to which the screw is attached for effective load transfer from spinal column to implant and to minimize the likelihood of slippage and/or gross failure, and (e) be strong enough to provide lasting and rigid fixation of the spine. Those skilled in the art will recognize that this list is not exhaustive, but is instead intended to illustrate some of the desirable characteristics of an ideal internal fixation system Other design criteria are also important, and some practicioners may consider some criteria so important that they might not even list others.

So far as is known, none of the above-listed internal fixation systems meets these criteria in every patient The disadvantages and limitations of currently available systems are made clear from reports in the literature of failure rates (failure of the device, not such complications as infection, phlebitis, seroma, neurologic deficit, etc.) as high as 25% (*see* R Roy-Camille, *et al.,* 203 Clin. Orthop. 7 (1986)), 11% *(see,* S.F. Heim and E.R Luque, Danek Plate and Screw System, *in* D.M Arnold and J.E. Lonstein (Eds.), 6 State of the Art Reviews - Spine: Pedicle Fixation of the Lumbar Spine 201-234 (Philadelphia: Nanley & Belfus, Inc.) 1992), 8% *(see,* R.M. Puno and J.A. Byrd III, Transpedicular Screw/Rod Fixation Using the Puno/Winter/Byrd (PWB) System, *supra),* and 2-7% D.M. Arnold and L.L. Wiltse, The Wiltse System of International Fixation for the Lumbar Spine, in D.M. Arnold and J.E. Lonstein (eds.), 6 State of the Art Reviews - Spine: Pedicle Fixation of the Lumbar Spine 55-82 (Philadelphia: Nanley & Belfus, Inc.) 1992.

The currently available systems have other limitations. By way of example, so far as is known, no currently available surgical implanted system can predictably treat rotoscoliosis. Further, no current available system is conveniently used in multiple level surgery. Multiple level surgery is a challenge for the surgeon because of the need to align the pedicle screws in multiple vertebrae while working under the heavy muscles of the back.

US Patent 5,743,907 disclose an apparatus to retain elements of the spine or pelvis in a desired special relationship. The arrangement includes an elongate rod and a plurality of transverse fixing elements. The fixing elements are secured to the rod. Each fixing element has an aperture to receive a fixing screw. In one embodiment the fixing screw is provided with a pair of wedge shaped washers which serve to hold the fixing screw at a desired angle relative to part of the element that defines an aperture through which the screw passes.

FR-2,697,993 discloses a further device for retaining vertebrae in predetermined positions. A screw is provided which has a hexagonal region lying immediately above a tapering threaded root. A washer is provided which can sit on the hexagonal region, the washer having a convex upper surface for angled engagement with a plate which has a concave recess surround a bore through which part of the screw may pass. In one embodiment the aperture passing through the washer is off-set from the centre of the washer.

There is therefore a need for improvement of such systems, and it is this improvement to which the present invention is directed.

According to this invention there is provided an internal spinal stabliser comprising a first elongate member, a second elongate member having an aperture therein, means for attaching said second elongate member to said first elongate member, a screw for passing through said aperture for affixing said second elongate member to the vertebra of a patient, and at least one washer having a cylindrical body provided with a passage for receiving said screw therethrough, one end of the cylindrical body being angled with respect to the side-walls of the cylinder to form a bearing surface, wherein said washer has a shoulder on the body to engage the aperture of the second elongate member to form an engagement between the washer and the aperture at a plurality of points within a common plane in any one of a plurality of relative rotated orientations of the body relative to the second elongate member.

A preferred embodiment of the present invention provides flexibility of placement, angulation, spacing and screw height for accommodating the pedicle screws of such systems.

The described embodiment of the invention provides a system which is universal in the sense that, although comprised of relatively few parts, it works with pedicle screws and laminar hooks, thereby providing even more flexibility and ease of use.

The preferred embodiment of the present invention also provides an internal spinal fixation system which avoids surgery under the heavy muscles of the back so that the surgery is simplified and there is more room for fusion of adjacent vertebrae in the lateral gutter.

The preferred embodiments of the present invention incorporate a connection between a spinal stabliser and pedicle screw comprising a washer having a shoulder for engaging a spinal stabliser, and means on the spinal stabiliser for engaging the washer, the shoulder on the washer and the engaging means of the spinal stabiliser co-operating to engage each other at a plurality of points within a common plane with the washer in any one of a plurality of relative rotational positions about an axis substantially perpendicular to the common plane relative to the spinal stabiliser. The washer defines a bearing surface and a passage extending through the washer, the pedicle screw extending through the passage and bearing against the bearing surface when the spinal stabiliser is affixed to a vertebral body.

The spinal stabiliser of the preferred embodiment of the present invention comprises an elongate member adapted to be affixed to a vertebra and defining a planar aperture. A washer is provided with a shoulder adapted to engage the elongate member adjacent the periphery of the aperture at any one of a plurality of relative rotational positions between the washer and the elongate member about a notional rotational axis which extends through the aperture, the washer having a passage therethrough, one end of the passage communication with the plane of the aperture and the other end of the passage being located at a bearing surface formed on the washer. A pedicle screw engages the bearing surface and extends through the passage and the aperture to engage the vertebral body of the vertebra, the axis of the passage intersecting the plane of the aperture at a first angle and the bearing surface at a second angle, at least one of the angles being acute, the plane of the aperture being inclined relative to the bearing surface so that the angle of inclination of the screw extending through the passage is adjusted in dependence upon the relative rotational position between the washer and the elongate member.

The invention will now be described, by way of example, with reference to the accompanying drawings in which:
FIGURE 1 is a partially schematic, dorsal view of a portion of the human spinal column having a first embodiment of a spinal stabiliser constructed in accordance with the teachings of the present invention surgically affixed thereto.
FIGURE 2 is a lateral view of the human spinal column having a spinal stabiliser of Figure 1 affixed thereto and showing the spinal column in phantom lines to show the positions of the pedicle screws used to affix the spinal stabiliser to the spinal column.
FIGURE 3 is a top plan view of a lumbar vertebra having the spinal stabiliser of Figures 1 and 2 affixed thereto and showing the vertebra in phantom lines to show the positions of the pedicle screws used to affix the spinal stabiliser to the spinal column.
FIGURE 4 is a top, perspective view of a washer, not being part of the invention.
FIGURE 5 is a top, perspective view of a washer, not being part of the invention.
FIGURE 6 is a bottom, perspective view of a third embodiment of a washer, constructed in accordance with the teachings of the present invention,
FIGURE 7 is a side elevational view of the washer of Figure 6,
FIGURE 8 is a bottom, perspective view of a fourth embodiment of a washer, constructed in accordance with the teaching of the present invention,
FIGURE 9 is a side elevational view of the washer of Figure 8,
FIGURE 10 is a perspective view of a second embodiment of the washer of Figure 8,
FIGURE 11 is a side elevational view of the washer of Figure 10.
Figure 12 is a partially exploded, perspective view of a portion of a second embodiment of a spinal stabilizer constructed in accordance with the teachings of the present invention.
Figure 13 is a side elevational view of a portion of the cross-bar of the spinal stabilizer of Fig. 12.
Figure 14 is a partially schematic, dorsal view of a portion of the human spinal column having a third embodiment of a spinal stabilizer constructed in accordance with the teachings of the present invention surgically affixed thereto.
Figure 15 is a perspective view of the cross-bar of the spinal stabilizer of Fig. 14.
Figure 16 is a dorsal view of a single lumbar vertebrae having a fourth embodiment of a cross-bar affixed thereto for use in connection with the spinal stabilizer of Fig. 14.
Figure 17 is an enlarged, exploded perspective view of the cross-bar of Fig. 16.
Figure 18 is a detailed, side elevational view of a portion of the cross-bar of Fig. 16.
Figure 19 is a partially schematic, dorsal view of a portion of a human spinal column having an embodiment of a spinal stabilizer constructed in accordance with the present invention affixed thereto.
Figure 20 is a perspective view of a portion of the spinal stabilizer of Fig. 19.
Figure 21 is a perspective, detailed view of a portion of the spinal stabilizer of Fig. 19.
Figure 22 is also a perspective, detailed view of a portion of the spinal stabilizer of Fig. 19.

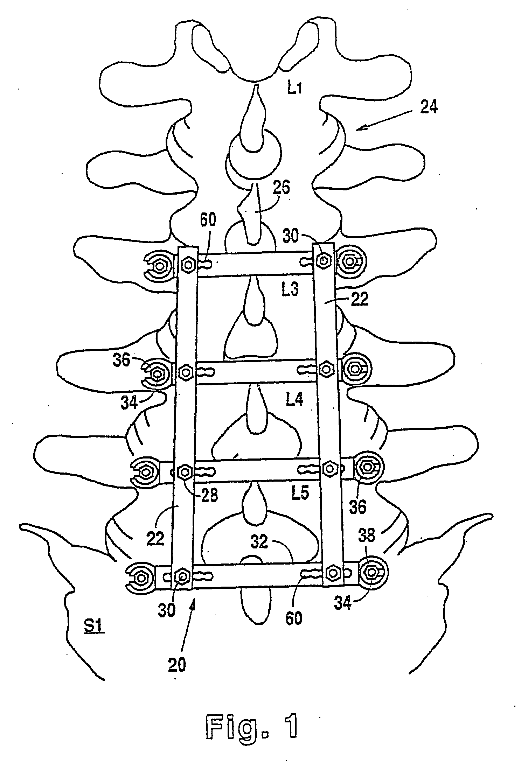

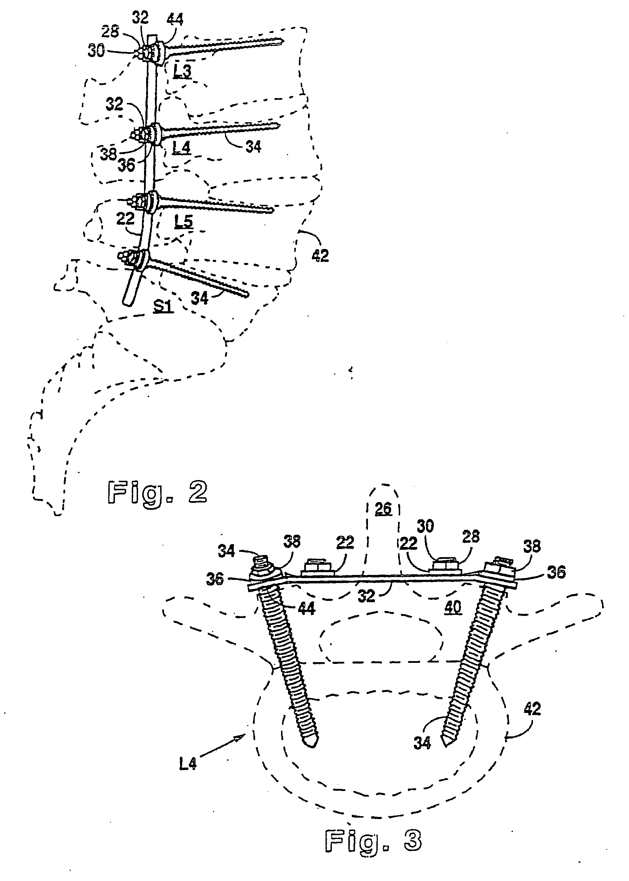

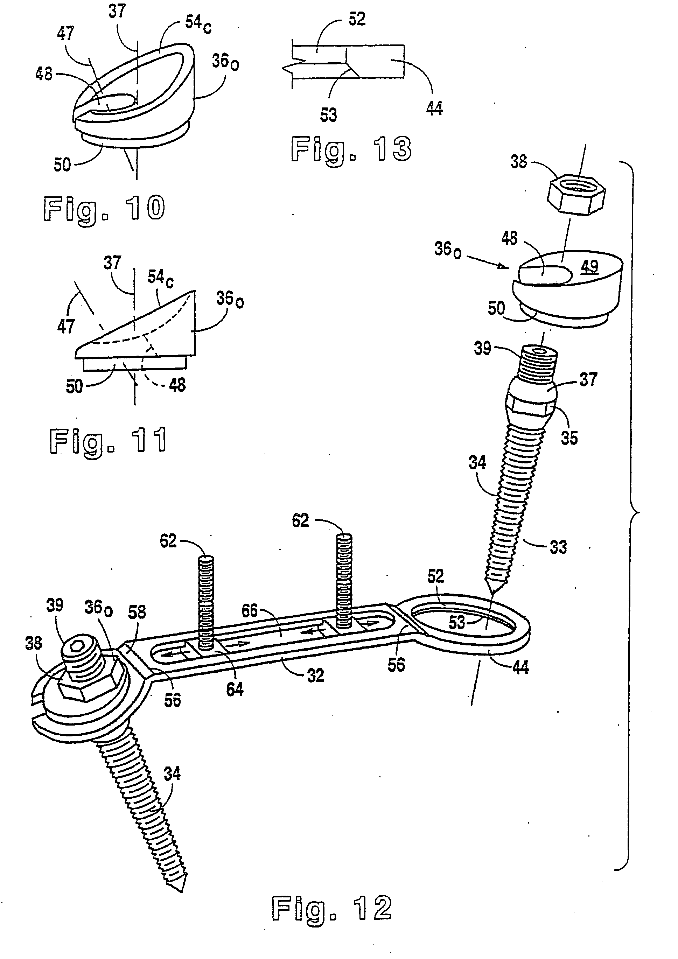

Referring now to the figures, a first embodiment of a spinal stabilizer constructed in accordance with the present invention is shown affixed to the spinal column in Figs. 1 and 2. This first embodiment, indicated generally at reference numeral 20, is comprised of two elongate members, or rods 22 oriented along the longitudinal axis of the spinal column 24 on either side of the spinous processes 26 of lumbar vertebrae L3-L5 and the first sacral vertebrae S1. Rods 22 are connected at the level of each vertebrae S1, L3-L5 by nut and-serew 28 and 30 to cross-bar, or plate, 32. The screws 30 are preferably of a type known in the art in which the portion of the screw threads projecting through nut 28 is broken off so as not to project any further from the nut 28 than needed. Each cross-bar/plate 32 is affixed to the corresponding vertebrae by a pedicle screw 34, washer 36, and nut 38, screws 34 being anchored in the pedicle 40 (see Fig. 3) of each vertebrae. Screws 34, shown in more detail in Fig. 12, are also of a type known in the art in which the bottom portion 33 is provided with threads for anchoring into the vertebrae, a head 35 with a rounded, or hemispherical upper surface 37, and an upper threaded portion 39 for threadably receiving the nut 38 and the portion of the threads projecting through nut 38 is broken off so as not to project any further through nut 38 than needed. A screw of this type is shown, for instance, in U.S. Patent No. 5,129,899.

As noted above, the need for secure anchorage of the screws 34 in the vertebrae, the lordosis of the spine and corresponding curve in rods 22 (best shown in Fig. 2), inward angle of the screws 34 (best shown in Fig. 3), different sizes, spacing, and shapes of the vertebral bodies 42, and many other factors. (including the particular pathology which the spinal fixation system is intended to address), require that almost every screw 34 must be oriented at a unique angle relative to rods 22. To illustrate, in Figs. 1 - 3, it can be seen that each screw is angled in three axes of a three-dimensional coordinate system (not shown) having its origin on the center axis of the spinal column 24. If the Y coordinate of the coordinate system is coincident with the center of the longitudinal axis of the spinal column 24 (such that +Y is anterior and -Y is posterior), the X coordinate is the lateral dimension, and the Z coordinate is orthogonal to the plane of the paper in Fig. 1 (such that +Z is ventral and -Z is dorsal), it can be seen in Fig. 2 that the positions of the tips of the screws 34 are defined by Y and Z coordinates. Reference to Fig. 3 (in which the Y coordinate of the hypothetical coordinate system runs in and out of the plane of the paper) illustrates that the positions of the tips of the same screws are also defined by an X coordinate.

Also in Fig. 3, it can be seen that the ends 44 of cross-bar 32 are angled downwardly, or ventrally (relative to the body of the patient), to accommodate the round shape of the body 42 of the vertebrae L4 to which the cross-bar 32 is affixed by screws 34. Although the cross-bar 32 need not be shaped in this fashion, this bend at the ends 44 of cross-bar 32 serves several advantages other than accommodating the shape of the vertebral body (for instance, reducing the height of the stabilizer in the direction of the Z coordinate of the three-dimensional coordinate system described above) and is therefore particularly adapted for use in the spinal fixation system of the present invention. However, the downward bends at the ends 44 also introduces yet another angle into the interface between the screw 34 and the cross-bar 32. As a result of the angle of the screw 34 and the bend at the end 44 of cross-bar 32, the longitudinal axis of screw 34 is unlikely to be perpendicular to cross-bar 32 at the connection between the screw 34 and cross-bar 32, and therefore unlikely to optimally transfer load from the spinal column 24 to the spinal stabilizer 20.

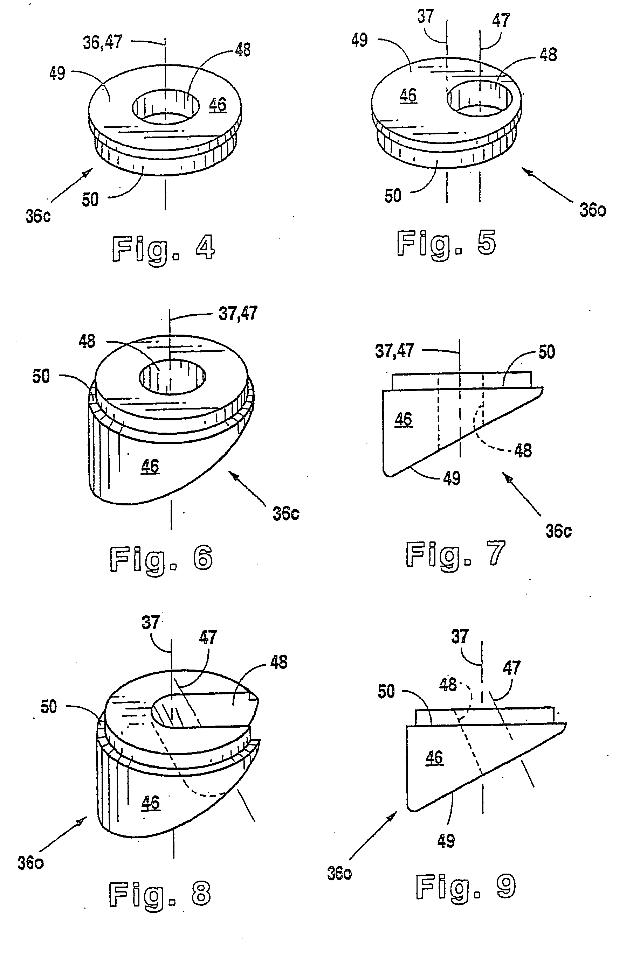

Figs. 4-9 show a plurality of washers 36 shaped and/or configured to provide optimal load transfer from the spinal column 24 to the spinal stabilizer 20 through pedicle screw 34. The washers 36_{c} and 36ₒ shown in Figs. 4 and 5, respectively, are comprised of a cylindrical body 46 having a longitudinal passage 48 therethrough for receiving the screw 34 for affixing the spinal stabilizer 20 to the vertebral body 42. The passage 48 through the body 46 of washer 36_{c} is centered on the longitudinal axis (represented by the phantom line 37, 47 in Fig. 4) of washer 36_{c} and the axis 47 of the passage 48 in the body 46 of washer 36ₒ (Fig. 5) is offset from the longitudinal axis 37 of the washer 36ₒ for a purpose to be described below. Each of the washers 36_{c} and 36ₒ is also provided with means formed on the body 46 for rotatably engaging the spinal stabilizer 20. This engagement means takes several forms; in the embodiment shown in Figs. 4-9, the stabilizer engaging means comprises a shoulder 50 formed on the body 46 of the washers 36_{c} and 36ₒ which rests on the cross-bar 32 (not shown) adjacent the periphery of the aperture 52 formed in the ends 44 of cross-bar 32 when the washer 36_{c} or 36ₒ is assembled to the cross-bar 32 in the manner described below.

Alternatively, and particularly in the case of the washers shown in Figs. 6-10 described below, the O.D. of the body 46 of washer 36 is provided with a groove and the washer is inserted with the angled end 49 (see below) up from beneath the aperture 52 in cross-bar 32 and rotated so that the groove engages and interacts with the periphery of the aperture 52 so as to limit the travel of the washer through the aperture 52. In another embodiment, the stabilizer engaging means takes the form of a plurality of projections projecting radially inwardly from the periphery of the aperture in the cross-bar for engaging a shoulder or slot formed on the O.D. of the body of the washer. In yet another embodiment, the washers are comprised of a resilient material such as a medical grade polymeric material which are provided with a groove formed on the O.D. thereof which is press-fit into the aperture 52 in cross-bar 32.

Embodiments of the washer 36 of the present invention are shown in Figs. 6-7 and 8-9. Again, the axis 47 of the passage 48 in the cylindrical body 46 of washer 36_{c} shown in Figs. 6 and 7 is centered on the longitudinal axis (represented by the phantom line 37 in Fig. 6) of washer 36_{c} and, when viewed from the end 49, the longitudinal passage 48 in the cylindrical body 46 of washer 36ₒ in Figs. 8 and 9 is offset from the center of the longitudinal axis (represented by the phantom line 37 in Fig. 8) of washer 36ₒ. Unlike the washers 36 shown in Figs. 4 and 5, the end 49 of each of the washers shown in Figs. 6-9 is angled at an angle other than 90° relative to the side walls of the cylindrically-shaped body 46, giving the body 46 a wedge shape. As a result of the angled end 49 of body 46, the axis 47 of the passage 48 through washer 36ₒ shown in Figs. 8-9 is not parallel to the longitudinal axis 37 of the body 46.

Like the washers 36 shown in Figs. 4-5, the washers 36 shown in Figs. 6-9 are provided with means for rotatably engaging an aperture in the spinal stabilizer 20 at any one of a plurality of relative rotational positions about a notional axis which extends through the aperture. As described above, in the embodiment shown, the stabilizer engaging means takes the form of a shoulder 50 formed on the body 46 of washers 36_{c} and 36ₒ which rests on the surface of cross-bar 32 adjacent the periphery of the aperture 52 formed in the ends 44 of cross-bar 32 when the washer 36_{c} or 36ₒ is assembled to the cross-bar 32 (the manner in which the shoulder functions is described below). Those skilled in the art will recognize from this disclosure that other shoulder engaging means such as are described above may be provided on the washers 36 to accomplish the intended function of allowing the washer 36 to rotate in engagement with cross-bar 32.

The bodies 46 of the washers 36 are described as being cylindrical to provide a basis for referring to the longitudinal axis of the washer 36 even though it will be recognized that the height of the right angle cylinder defined by the washers of Figs. 4 and 5 is minimal since the washers shown in Figs. 4 and 5 are accurately described as ''flat washers." However, as set out above, one end 49 (defining the bearing surface against which the screw 34 is tightened to affix the spinal stabilizer to the vertebra) of the bodies 46 of the washers 36 shown in Figs. 6-9 is angled relative to the side walls of the cylindrically-shaped body 46 of washer 36. In the embodiments shown in Figs. 4 and 5, the end 49 is angled at an angle of approximately 90° such that the washers shown in those figures are flat, but the end 49 of the bodies 46 of the washers 36 shown in Figs. 6-9 is angled relative to the side walls at an angle other than 90° such that the bodies 46 of washers 36 shown in those figures have a substantial vertical dimension and are wedge-shaped rather than flat. A variety of angles may be utilized to advantage, but angles (relative to the side walls of the cylindrically-shaped body 46) ranging from about 7.5° to about 30° have generally proven to be adequate to provide a full range of adjustability. It will also be recognized by those skilled in the art that although the washer of the present invention is defined as being cylindrical, all that is required is that the washer be capable of being rotated to a plurality of positions relative to the cross-bar of the spinal stabilizer. A plurality of positions can be obtained with, for instance, a square (or octagonal or triangular) aperture in the cross-bar and a similarly-shaped washer since the washer could then be placed in as many as four (or eight or three) different relative positions about the notional axis which is substantially perpendicular to the plane of the aperture. All such embodiments are contemplated by the use of the word "cylindrical" in describing the washer of the present invention.

Referring now to Figs. 10 and 11, an alternative embodiment of the washer of the present invention is shown. The washer 36 shown in Figs. 11 and 12 is similar to the washers shown in Figs. 6-9, but is provided with a concave end 49 for interaction with a nut 38 (not shown) having a convex lower surface. When tightened onto the pedicle screw 34, the concave end 49 provides an even greater range of angles and adjustability of placement of the screw 34 relative to the central axis of the spinal column. Because the end 49 is the end surface of the body 46 comprising washer 36 against which the pedicle screw 34 bears, it is referred to herein as the bearing surface 49 of washer 36.

As best shown in Figs. 3 and 12, washers 36 rotatably engage cross-bar 32 at the ends 44. As described above, rotatable engagement is accomplished by resting the shoulders 50 of washers 36 comprising the preferred stabilizer engaging means on the margins of the apertures 52 at the ends of cross-bars 32. During the surgical procedure, the surgeon selects either
a flat washer with a longitudinal passage coincident with the center of the longitudinal axis of the washer (Fig. 4),
a flat washer with a passage offset from the center of the longitudinal axis of the washer (Fig. 5),
a wedge-shaped washer with a passage coincident with the center of the longitudinal axis of the washer (Figs. 6-7), or
a wedge-shaped washer with a passage offset from the center of the longitudinal axis of the washer (Figs. 8-9),
and then rotates the body 46 of the washer 36 selected in the aperture 52 to provide infinite adjustability of the linkage between the cross-bar 32 and screw 34, regardless of the angle and position of the screws 34 in the pedicle 40 of each vertebrae and regardless of the shape, size, or pathology of the vertebrae and/or pedicle. The washer selected is preferably the washer which, by its shape and ability to be rotated, locates the passage therethrough in the proper position for receiving the screw 34 while maintaining an angle of approximately 90° between the longitudinal axis of the screw 34 and the bearing surface 49 of the washer against which the nut 38 bears when tightened to effectively transfer load from the spinal column 24 to the implant 20. It will be noted that when the screw 34 engages the end 49 of washer 36 and extends through the passage 48 and the aperture 52 to engage the vertebra, the axis 47 of passage 48 intersects the plane of the aperture at a first angle and, depending on whether the end 49 is inclined, or angled, the rotational position of the washer relative to the spinal stabilizer, may intersect the end 49 at a second angle, but that at least one of the first or second angles must be an acute angle.

To facilitate assembly of the cross-bar 32 to the screws 34, one end 44 of cross-bar 32 may be provided with a gap or break 45 through which the portion of the screw 34 protruding from the pedicle is maneuvered. Also, because the angle between the underside of cross-bar 32 and the longitudinal axis of screw 34 is unlikely to be a 90° angle and/or the screw 34 may be positioned close to the inside margin of the aperture 52 in cross-bar 32, the upper surface 37 of the head 35 of screw 34 is rounded and the inside surface of the margin of the apertures 52 in cross-bar 32 is beveled as shown at reference numeral 53 in Fig. 13. In another embodiment (not shown), the head of screw 34 is sized so as to contact the margin of aperture 52 in cross-bar 32, the bevel 53 and complementary rounded upper surface 37 of the screw 34 helping to center the longitudinal axis of aperture 52 in cross-bar 32 on the longitudinal axis of screw 34.

Fig. 12 shows the interaction and adjustability of the spinal implant of the present invention by showing one screw 34 to which a nut 38 is tightened against the wedge-shaped washer 36ₒ of Figs. 8 and 9 with the washer 36ₒ having been rotated relative to cross-bar 32 in the common plane formed by the cooperating engaging means on the washer and the eross-bar 32 of the spinal stabilizer so that the screw 34 is angled anteriorally (with reference to the patient) while also being angled inwardly (relative to the central axis of the spinal column 24). A flat washer 36ₒ is shown on the other side of the cross-bar 32 which has been rotated so that the screw 34 is not centered in the aperture 52 of cross-bar 32 but the inward angle of the screw resulting from the downward bend at the ends 44 of cross-bar 32 is maintained. Cross-bar 32 is shown with an optional nipple 56 forming a stop surface 58 near the apertures 52 therethrough which acts, by engagement of the O.D. of the washer 36, to restrain any tendency of the washer 36 to move inwardly from the ends 44 of cross-bar while the nut 38 is being tightened against the washer 36 when in place in the aperture 52 in cross-bar 32.

Referring briefly again to Fig. 1, the portion of cross-bar 32 intermediate the ends 44 is provided with a plurality of nested slots 60 of a type known in the art (*see,* for instance, U.S. Patent No. 4,696,290 and the so-called VSP spinal fixation system described in J.W. Brantigan, et aL, Posterior lumbar interbody fusion technique using the variable screw placement spinal fixation system, in D.M. Arnold and J.E. Lonstein (Eds.), 6 State of the Art Reviews - Spine: Pedicle Fixation of the Lumbar Spine 201-234 (Philadelphia: Nanley & Belfus, Inc.) 1992, for precise placement of the screws 30 securing the rods 22 to the cross-bars 32 along the longitudinal axis of cross-bar 32. The screws 30 for securing the rods 22 to the cross-bars 32 are of the above-described type known in the art in which the portion of the screw which extends above the top surface of the nut 28 is broken off after the nut is tightened.

By comparison to Fig. 1, it can be seen that in the embodiment shown in Fig. 12, the attachment between rods 22 and cross-bars 32 is accomplished by tightening nuts 28 to the posts 62 integrally mounted to the plate 64 which moves from side to side along the longitudinal axis of cross-bar 32 in the slot 66 formed therein. The plate 64 is comprised of a flat portion (not visible in Fig. 12 because of the perspective in the figure) which extends under the cross-bar and which is tightened against the underside of cross-bar 32 when the nut 28 is tightened against a rod 22 to prevent further side to side movement of the plate 64 and post 62. Before tightening the nut 28, the plate is moved by the surgeon to the position which allows precise alignment of the rod 22 with the cross-bar 32.

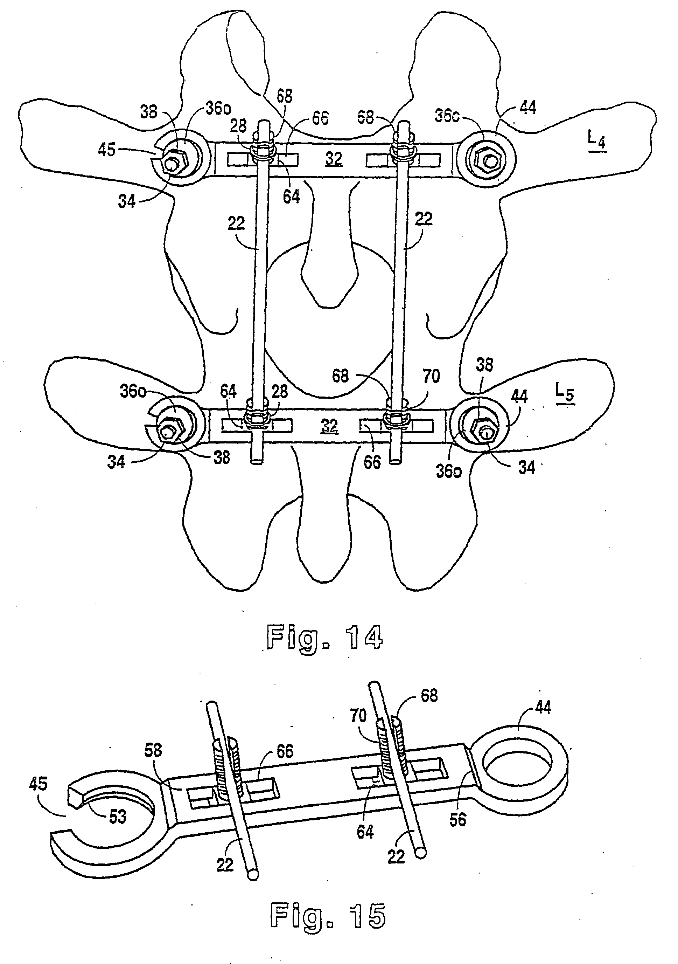

In the embodiment shown in Figs. 14-15, the rods 22 are of a type known in the art such as those available from MOSS® Miami (Cat. No. 1745-70, -72, and -74) which are attached to cross-bars 32 by U-shaped connectors 68 having threads 70 formed on the outside surfaces thereof. For purposes of convenience, the rods 22 may be referred to generically as first elongate members and the cross-bars 32 are referred to a second elongate members. Connectors 68 are integrally mounted to a plate 64 having a construction similar to that of the so-called axial connectors available from MOSS® Miami (Cat. No. 1745-61 and -62), e.g., two halves (not shown) with threaded posts and nuts for connecting the halves on the top and bottom of the cross-bar 32 to clamp the cross-bar 32 and prevent side to side movement of the plate 64 in the slot 66 in cross-bar 32 in which the plate 64 moves. Alternatively, the plate 64 is provided with a portion extending under cross-bar 32 which is tightened against the underside of cross-bar 32 when the nut 28 is tightened on connector 68 to resist further side to side movement. Those skilled in the art will recognize from this description of the connectors 68 and plates 64 that a similar arrangement may be used in place of the nested slots 60 in the cross-bar 32 of the embodiment shown in Fig. 1 wherein the threaded posts 62 are replaced by connectors 68 for precise lateral placement of the point at which the rods 22 are attached to cross-bars 32. In such an embodiment, connectors 68 are provided with a head for engaging the underside of the cross-bar 32 in the same manner as the screws 30.

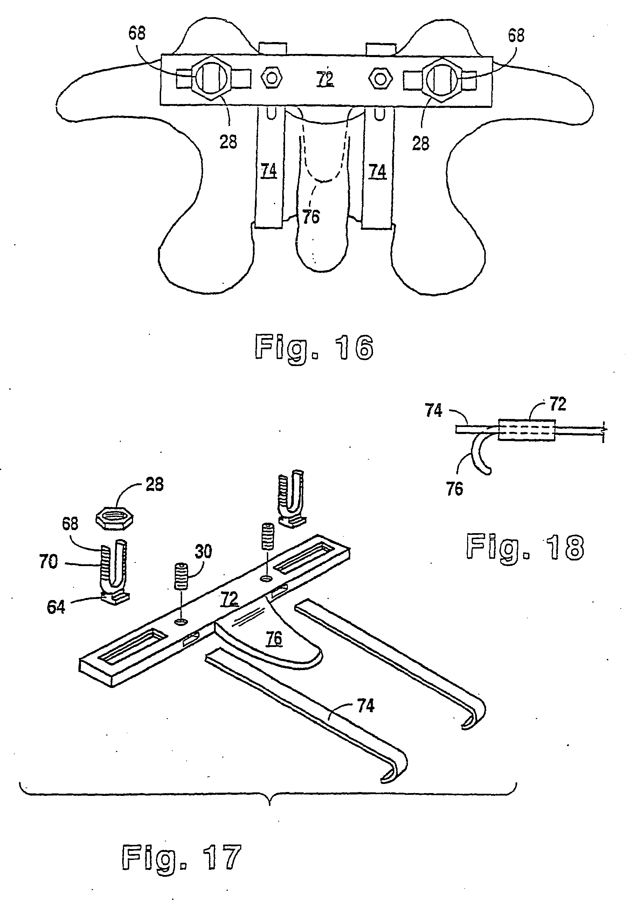

Referring now to Figs. 16-18, the second elongate member comprises a cross-bar 72 of a type modified for use in connection with the embodiment of the spinal stabilizer of the present invention shown in Figs. 14-15. The cross-bar 72 is provided with hooks 74 for engaging the lamina of the vertebra and a retainer 76 which is curved so as to extend under the lamina to which cross-bar 72 is to be affixed. The hooks 74 extend through a slot (not numbered for the sake of clarity) formed at approximately a right angle to the longitudinal axis of cross-bar 72 and are extended in and out of that slot until they are adjusted so as to tightly engage the posterior margin of the lamina and then set in that position by tightening the set screw 78 provided in cross-bar 72 for that purpose. U-shaped connectors 68 mounted on plates 64 as described above are provided for connecting to a rod 22 as shown in Figs. 14-15.

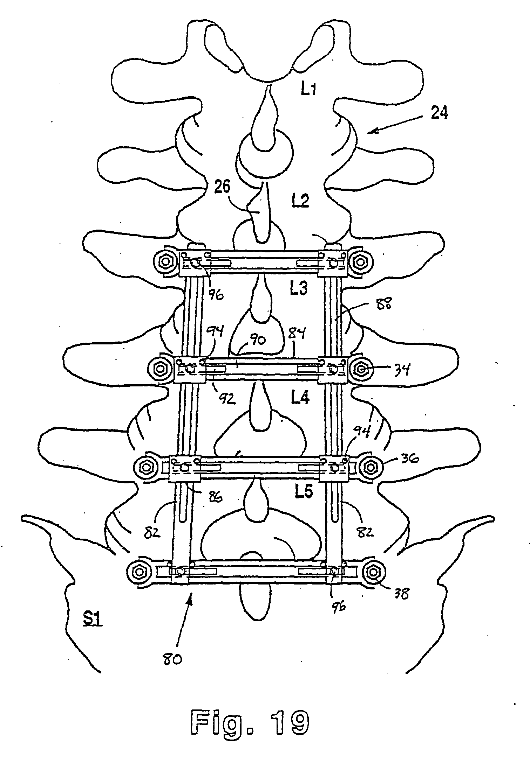

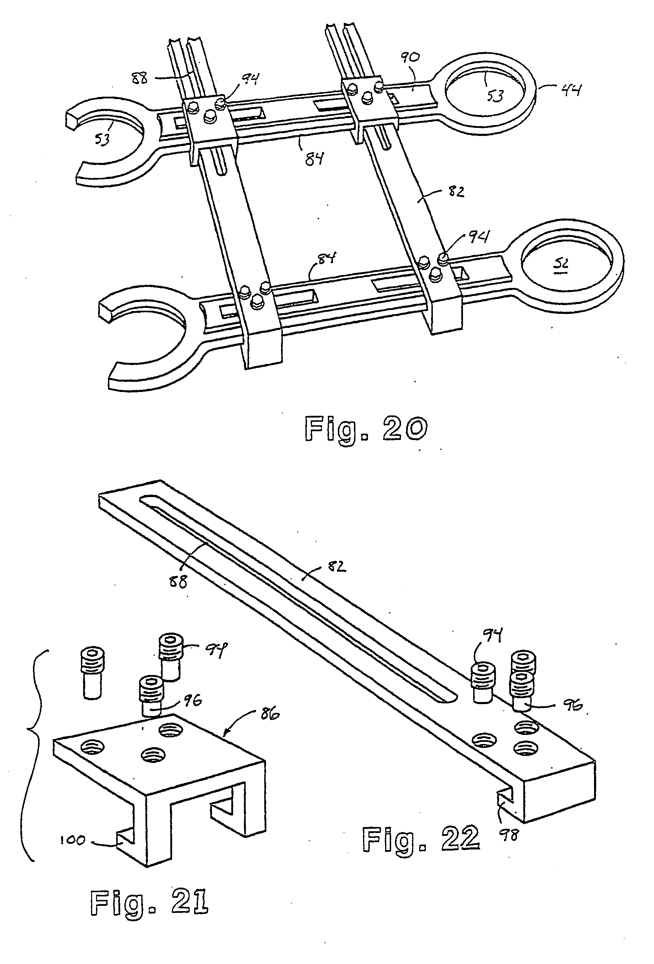

Another embodiment of a spinal stabilizer constructed in accordance with the present invention is shown in Figs. 19-22. In this embodiment, indicated generally at reference numeral 80, both the rods and cross-bars of the spinal stabilizer are formed in the shape of flat, elongate members, and are therefore referred to as first and second elongate members 82 and 84, respectively. First and second elongate members 82 and 84 are attached to each other at an angle of approximately 90° by the interaction of the brackets 86, slots 88, raised ridges 90, gutters 92, guide screws 94, and set screws 96. In more detail, the first and second elongate members 82 and 84 are assembled to each other by placing a second elongate member 84, which functions in the manner of the cross-bar 32 in the embodiments shown in Figs. 1-3 and 12-15, into the "L" 98 of first elongate member 82 and tightening the guide screws 94 and set screw 96 until the screws 94 and 96 engage the margins of the raised ridge 90 and gutter 92 formed on the second elongate member 84. In this manner, the second elongate member 84 is affirmatively connnected to the first elongate member 82, but the second elongate member is movable along its longitudinal axis relative to first elongate member 82. When the surgeon has placed the second elongate member 84 in the desired location, the set screw 96 is tightened in the gutter 92 to force the back side of second elongate member 84 against the inside of the "L" 98 of first elongate member 82 to retain the second elongate member 84 in that selected position relative to first elongate member 82 and prevent further sliding movement of second elongate member 84 along its longitudinal axis relative to first elongate member 82.

The mounting bracket 86 is then assembled to first elongate member 82 with the set screw 96 riding in the slot 88 of first elongate member 82 and another second elongate member 84 is inserted between the inside surface of the tabs 100 straddling the first elongate member 82 and the underside of the first elongate member 82. Guide screws 94 are then tightened sufficiently to retain the bracket and second elongate member 84 to first elongate member 82 and the second elongate member is slidably adjusted up and down first elongate member 82 to the desired location by the surgeon. Second elongate member 84 is slid back and forth along its longitudinal axis to the desired location relative to the patient's spinal column as described above and the guide and set screws 94, 96 are tightened to affirmatively retain the second elongate member 84 is the selected position relative to first elongate member 82 as described above. Washers of the appropriate shape and size are then selected as required to provide a connection between pedicle screw 34 (not shown) and the spinal stabilizer 80 which provides optimal load transfer between vertebrae and spinal stabilizer. A particular advantage of the embodiment shown in Figs. 19-22 is its low "profile." In other words, when affixed to the vertebrae comprising a patient's spinal column, the dorsal extension of the embodiment shown in Figs. 19-22 is minimized.

Although described in terms of the presently preferred embodiment shown in the figures, those skilled in the art will recognize from this description that changes can be made to the component parts of the present invention without changing the manner in which those component parts function to achieve their intended result For instance, the present invention is equally adaptable to a spinal fixation system which is comprised of rods on either side of the processes of the vertebrae which may or may not be connected by a cross-bar or a system comprised of a single rod down the dorsal aspect of the spinal column after removal of the dorsal processes rather than the ladder-type system shown in the figures. All such changes, and the others known to those skilled in the art, are intended to fall within the scope of the following claims.

## Claims

1. An internal spinal stabiliser comprising a first elongate member (22), a second elongate member (32) having an aperture (52) therein, means (28, 30) for attaching said second elongate member to said first elongate member, a screw (34) for passing through said aperture for affixing said second elongate member to the vertebra of a patient, and at least one washer (36) having a cylindrical body (46) provided with a passage (48) for receiving said screw (34) therethrough, one end (49) of the cylindrical body being angled with respect to the side-walls of the cylinder to form a bearing surface (49), **characterised in that** said washer has a shoulder (50) on the body (46) to engage the aperture of the second elongate member to form an engagement between the washer and the aperture at a plurality of points within a common plane in any one of a plurality of relative rotated orientations of the body relative to the second elongate member.

## Patentansprüche

1. Interne Wirbelsäulenstütze, die ein erstes längliches Teil (22), ein zweites längliches Teil (32) mit einer Öffnung (52) darin, Mittel (28, 30) zur Befestigung besagten zweiten länglichen Teils an besagtem ersten länglichen Teil, eine Schraube (34) zum Durchgang durch besagte Öffnung zur Fixierung besagten zweiten länglichen Teils am Wirbel eines Patienten und wenigstens eine Unterlegscheibe (36) mit einem zylindrischen Körper (46), der mit einem Durchlaß (48) zur Aufnahme besagter Schraube (35) dorthindurch versehen ist, umfaßt, wobei ein Ende (49) des zylindrischen Körpers in Bezug auf die Seitenwände des Zylinders abgewinkelt ist, um eine Auflagefläche (49) zu bilden, **dadurch gekennzeichnet, daß** besagte Unterlegscheibe eine Schulter (50) auf dem Körper (46) aufweist, um mit der Öffnung des zweiten länglichen Teils in Eingriff zu kommen, um einen Eingriff zwischen der Unterlegscheibe und der Öffnung an mehreren Punkten in einer gemeinsamen Ebene in jeder von mehreren relativ gedrehten Ausrichtungen des Körpers relativ zum zweiten länglichen Teil zu bilden.

## Revendications

1. Un stabilisateur spinal interne, comprenant un premier organe (22) allongé, un deuxième organe (32) allongé ayant en son sein une ouverture (52), des moyens (28, 30) pour attacher ledit deuxième organe allongé audit premier organe allongé, une vis (34) pour passer à travers ladite ouverture pour fixer ledit deuxième organe allongé à la vertèbre d'un patient, et au moins une rondelle (36), ayant un corps (46) cylindrique muni d'un passage (48) pour recevoir ladite vis (34) à travers lui, une extrémité (49) du corps cylindrique étant inclinée par rapport aux parois latérales du cylindre pour former une surface de portée (42), **caractérisé en ce que** ladite rondelle comprend, sur le corps (46), un épaulement (50), devant venir en prise avec l'ouverture du deuxième organe allongé pour établir une mise en prise entre la rondelle et l'ouverture en une pluralité de points dans un plan commun, dans l'une quelconque d'une pluralité d'orientations relatives en rotation du corps par rapport au deuxième organe allongé.
